# EUROPEAN PATENT APPLICATION

(11) **EP 3 722 289 A1**
(43) Date of publication of application: **14.10.2020**
(21) Application number: 18886513.3
(22) Date of filing: 04.12.2018
(51) Int. Cl.: C07D 401/04, C07D 213/89

(54) **METHOD FOR PRODUCING 2-SUBSTITUTED-3-ETHYLSULFONYL PYRIDINE COMPOUND AND LIKE**

(30) Priority: 05.12.2017 JP 2017233133; 18.09.2018 JP 2018173447
(71) Applicant: Sumitomo Chemical Company Limited, Tokyo 104-8260 (JP)
(72) Inventor: HAGIYA, Koji, Takarazuka-shi Hyogo 665-8555 (JP); ISHIKAWA, Junichi, Takarazuka-shi Hyogo 665-8555 (JP)
(74) Representative: Vossius & Partner Patentanwälte Rechtsanwälte mbB
(86) International application number: PCT/JP2018/044474
(87) International publication number: WO 2019/111869

(57) **Abstract**

The present invention provides an intermediate compound for preparing a 2-substituted-3-ethylsulfonylpyridine compound, and a novel process for preparing the 2-substituted-3-ethylsulfonylpyridine compound. The present invention provides also a process for preparing a compound represented by formula (2), which comprises a step (a): a step of reacting of 3-ethylsulfonylpyridine N-oxide with a compound represented by formula (1) (wherein X represents a chlorine atom etc., Q represents a C1-3 alkoxy group optionally substituted with one or more fluorine atoms, etc.) in the presence of one or more compounds selected from Group P, a carboxylic acid or a carboxylate salt, a base, and a palladium compound or a nickel compound in a solvent to obtain a compound represented by formula (2), Group P: a group consisting of the following compounds: a compound represented by formula (4): R₃P (wherein R represents an alkyl group having 1 to 6 carbon atoms, etc.), etc.; as well as a process for preparing a compound represented by formula (3), which comprises the step (a) and a step (b) : a step of subjecting the compound represented by formula (2) to a reduction reaction to obtain the compound represented by formula (3).

## Description

### TECHNICAL FIELD

This application claims priority to and the benefit of Japanese Patent Application Nos. 2017-233133 filed December 5, 2017, and 2018-173447 filed September 18, 2018, the entire contents of which are incorporated herein by reference.

The present invention relates to an intermediate compound for preparing a 2-substituted-3-ethylsulfonylpyridine compound and a process for preparing the 2-substituted-3-ethylsulfonylpyridine compound.

### BACKGROUND ART

2-Substituted-3-ethylsulfonylpyridine compounds have been known as an active ingredient of pest control agent (see Patent Document 1).

### CITATION LIST

### PATENT DOCUMENT

Patent Document 1: WO 2017/065228

### SUMMARY OF THE INVENTION

### (PROBLEMS TO BE SOLVED BY INVENTION)

An object of the present invention is to provide an intermediate compound for preparing a 2-substituted-3-ethylsulfonylpyridine compound, and a novel process for preparing the 2-substituted-3-ethylsulfonylpyridine compound.

### (MEANS TO SOLVE PROBLEMS)

The inventors have found out that 3-ethylsulfonylpyridine N-oxide and a compound represented by formula (1): 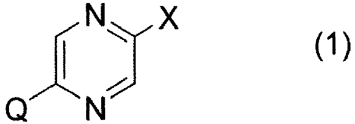 (wherein X represents a chlorine atom or a bromine atom, Q represents a C1-3 alkoxy group optionally substituted with one or more fluorine atoms, a fluorine atom, a chlorine atom, or a bromine atom.)
are reacted under a coupling reaction condition to proceed with a selective reaction at 2-position of 3-ethylsulfonylpyridine N-oxide to obtain 2-substituted-3-ethylsulfonylpyridine N-oxide in a high yield, and further that the obtained 2-substituted-3-ethylsulfonylpyridine N-oxide can be easily converted into a 2-substituted-3-ethylsulfonylpyridine, and thus have completed the present invention.

That is, the present invention includes the followings.
[1] A process for preparing a compound represented by the below-mentioned formula (2), which comprises a step (a) : a step of reacting of 3-ethylsulfonylpyridine N-oxide with a compound represented by formula (1): 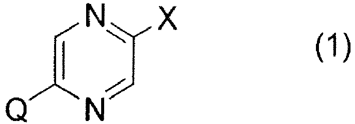 (wherein X represents a chlorine atom or a bromine atom, Q represents a C1-3 alkoxy group optionally substituted with one or more fluorine atoms, a fluorine atom, a chlorine atom, or a bromine atom.)
   in the presence of one or more compounds selected from Group P, a carboxylic acid or a carboxylate salt, a base, and a palladium compound or a nickel compound in a solvent to obtain a compound represented by formula (2): 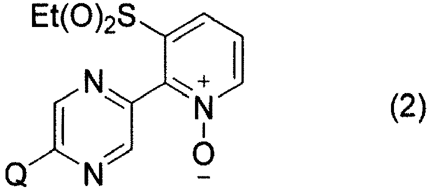 (wherein Q is the same as defined above.),
   Group P: a group consisting of the following compounds:
   a compound represented by formula (4):

      R₃P (4)

      (wherein R represents an alkyl group having 1 to 6 carbon atoms or an alkenyl group having 3 to 6 carbon atoms),
   a compound represented by formula (5):

      R₃PH⁺Z⁻ (5)

      (wherein R is the same as defined above, and Z⁻ represents an anion.),
   a compound represented by formula (6):

      R₂P(CH₂)ₙPR₂ (6)

      (wherein R is the same as defined above, and n is an integer of 1 to 4.), and
   a compound represented by formula (7):

      R₂PH⁺(CH₂)ₙPR₂H⁺2Z⁻ (7)

      (wherein R, n and Z⁻ are the same as defined above.).
[2] The process according to [1] wherein the compound selected from Group P is one or more compounds selected from 2-butenyl(di-t-butyl)phosphine, trimethylphosphine, 2-butenyl(di-t-butyl)phosphonium tetrafluoroborate, methyl(di-t-butyl)phosphine, methyl(di-t-butyl)phosphonium tetrafluoroborate, trimethylphosphonium tetrafluoroborate, 1,4-bis(di-t-butylphosphino)butane, or 1,4-bis(di-t-butylphosphino)butane bistetrafluoroborate.
[3] The process according to [1] or [2] wherein the step (a) is conducted in the presence of copper or copper compound.
[4] The process according to [3] wherein the copper is copper powder, and the copper compound is copper(I) oxide.
[5] A process for preparing a compound represented by the below-mentioned formula (3): 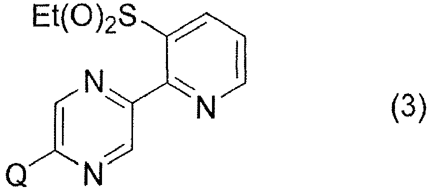 (wherein Q represents a C1-3 alkoxy group optionally substituted with one or more fluorine atoms, a fluorine atom, a chlorine atom, or a bromine atom.)
   , which comprises a step (a) according to any one of [1] to [4] and the below-mentioned step (b),
   a step (b) : a step of subjecting the compound represented by formula (2): 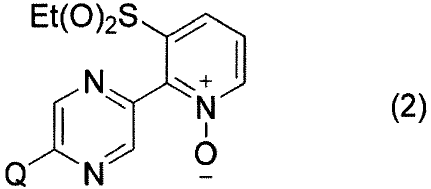 (wherein Q is the same as defined above.)
   to a reduction reaction to obtain the compound represented by formula (3).
[6] The process according to [5] wherein in the step (b), the reduction reaction is conducted by using hydrogen gas.
[7] The process according to any one of [1] to [6] wherein X represents a chlorine atom, and Q represents a 2,2,3,3,3-pentafluoropropoxy group.
[8] A 3-ethylsulfonylpyridine N-oxide.
[9] A compound represented by formula (2): 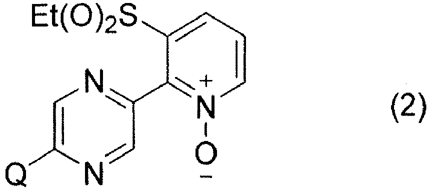 (wherein Q represents a C1-3 alkoxy group optionally substituted with one or more fluorine atoms, a fluorine atom, a chlorine atom, or a bromine atom.).
[10] A 2-[3-(ethylsulfonyl)-1-oxide-2-pyridinyl]-5-(2,2,3,3,3-pentafluoropropoxy)pyrazine.
[11] A 2-[3-(ethylsulfonyl)-1-oxide-2-pyridinyl]-5-chloropyrazine.

### [EFFECT OF INVENTION]

The present invention can provide an intermediate for preparing the 2-substituted-3-ethylsulfonylpyridine compound, and a process for preparing the 2-substituted-3-ethylsulfonylpyridine compound.

### MODE FOR CARRYING OUT THE INVENTION

Hereinafter, the present invention is explained in detail.

Step (a) is explained.

In the step (a), 3-ethylsulfonylpyridine N-oxide is reacted with a compound represented by formula (1) in the presence of one or more compounds selected from Group (P), a carboxylic acid or a carboxylate salt, a base, and a palladium compound or a nickel compound, in a solvent, to obtain a compound represented by formula (2).

The substituents expressed by symbols through the formula (1) to the formula (8) as used herein are explained below.

Et represents an ethyl group.

Examples of a C1-3 alkoxy group optionally substituted with one or more fluorine atoms as a symbol Q include methoxy group, ethoxy group, propoxy group, isopropoxy group, trifluoromethoxy group, difluoromethoxy group, 2,2,2-trifluoroethoxy group, pentafluoroethoxy group, 2,2,3,3,3-pentafluoropropoxy group, and 1,1,1,3,3,3-hexafluoroisopropoxy group.

Examples of an alkyl group having 1 to 6 carbon atoms as a symbol R include methyl group, ethyl group, propyl group, isopropyl group, butyl group, isobutyl group, t-butyl group, pentyl group, cyclopentyl group, hexyl group, and cyclohexyl group.

Examples of an alkenyl group having 3 to 6 carbon atoms as a symbol R include propenyl group, 2-butenyl group, 3-butenyl group, 3-methyl-2-butenyl group, 4-pentenyl group, and 5-hexenyl group.

Z⁻ represents an anion such as tetrafluoroborate.

Preferred examples of the solvent include aprotic organic solvent.

The aprotic organic solvent represent an organic solvent that does not contain any proton-containing groups such as hydroxide group (-OH), an amino group, and a carboxyl group (-COOH) in a molecule and also is capable of dissolving the 3-ethylsulfonylpyridine N-oxide and the compound represented by formula (1).

Examples of the aprotic organic solvents include ethers such as non-cyclic ether and cyclic ether, aromatic hydrocarbons, as well as aliphatic hydrocarbons.

Examples of the non-cyclic ether include diethyl ether, diisopropyl ether, ethylene glycol dimethyl ether, and diethylene glycol dimethyl ether. Examples of the cyclic ether include 1,4-dioxane, and tetrahydrofuran. Examples of the aromatic hydrocarbons include benzene, toluene, xylene, and mesitylene. Examples of the aliphatic hydrocarbons include hexane, heptane, and cyclohexane.

In the terms of solubility of the 3-ethylsulfonyl pyridine N-oxide and the compound represented by formula (1), toluene, xylene, mesitylene, diethyl ether, diisopropyl ether, ethylene glycol dimethyl ether, diethylene glycol dimethyl ether, 1,4-dioxane, and tetrahydrofuran are preferred. As needed, two or more kinds of the aprotic organic solvents may be used in combination with each other, and specific examples of the combined organic solvents include a mixed solvent of tetrahydrofuran and toluene, and a mixed solvent of ethylene glycol dimethyl ether and toluene.

The amount used of the solvent is within the range of usually 1 to 50 parts by weight, and preferably 1 to 20 parts by weight, as opposed to 1 part by weight of the compound represented by formula (1).

The palladium compound represents a compound in which any atoms other than palladium is/are bounded to a palladium, and includes preferably palladium(0) complex, and palladium(II) complex.

Examples of the palladium(0) complex include a complex in which dibenzylideneacetone coordinates to 0(zero)-valent palladium, namely, dibenzylideneacetone palladium(0) complex. Specific examples thereof include tris(dibenzylideneacetone)dipalladium(0), tris(dibenzylideneacetone)dipalladium(0) chloroform adduct, and bis(dibenzylideneacetone)palladium(0).

Examples of the palladium(II) complex include palladium carboxylate such as palladium(II) acetate, palladium(II) trifluoroacetate, palladium(II) acetylacetonate; halogenated palladium such as palladium(II) chloride, palladium(II) bromide and palladium iodide(II); and halogenated palladium complex such as allylpalladium(II) chloride dimer, bis(2-metyallyl)palladium(II) chloride dimer, dichloro(1,5-cyclooctadiene)palladium(II), dichlorobis(acetonitrile)palladium(II), and dichlorobis(benzonitrile)palladium(II). Among them, palladium(II) chloride, palladium(II) bromide, and palladium(II) acetate are preferred.

The amount used of the palladium compound is within the range of usually 0.00001 to 0.8 moles, and preferably 0.00002 to 0.2 moles, as opposed to 1 mole of the compound represented by formula (1).

The nickel compound represents a compound wherein any atoms other than nickel is/are bounded to a nickel, and includes preferably nickel(0) complex and nickel(II) complex.

Examples of the nickel(0) complex include bis(1,5-cyclooctadiene)nickel(0).

Examples of the nickel(II) complex include nickel carboxylate salts such as nickel(II) acetate, nickel(II) trifluoroacetate, nickel(II) acetylacetonate; and halogenated nickel such as nickel(II) chloride, nickel(II) bromide, and nickel(II) iodide. Among them, nickel(II) chloride, nickel(II) bromide, and nickel(II) acetate are preferred.

The amount used of the nickel compound is within the range of usually 0.0001 to 0.8 moles, and preferably 0.0002 to 0.2 moles, as opposed to 1 mole of the compound represented by formula (1).

Specific examples of the compound represented by formula (1) include 2-chloro-5-methoxypyrazine, 2-chloro-5-ethoxypyrazine, 2-chloro-5-propoxypyrazine, 2-chloro-5-isopropoxypyrazine, 2-chloro-5-isopropoxypyrazine, 2-chloro-5-(trifluoromethoxy)pyrazine, 2-chloro-5-(2,2,2-trifluoroethoxy)pyrazine, 2-chloro-5-(1,1,2,2-tetrafluoroethoxy)pyrazine, 2-chloro-5-(2,2,3,3,3-pentafluoropropoxy)pyrazine, 2-chloro-5-(2,2,3,3-tetrafluoropropoxy)pyrazine, 2-chloro-5-(3,3,3-trifluoropropoxy)pyrazine, 2-chloro-5-(1,1,1,3,3,3-hexafluoroisopropoxy)pyrazine, 2-bromo-5-methoxypyrazine, 2-bromo-5-ethoxypyrazine, 2-bromo-5-propoxypyrazine, 2-bromo-5-isopropoxypyrazine, 2-bromo-5-(trifluoromethoxy)pyrazine, 2-bromo-5-(2,2,2-trifluoroethoxy)pyrazine, 2-bromo-5-(1,1,2,2-tetrafluoroethoxy)pyrazine, 2-bromo-5-(2,2,3,3,3-pentafluoropropoxy)pyrazine, 2-bromo-5-(2,2,3,3-tetrafluoropropoxy)pyrazine, 2-bromo-5-(3,3,3-trifluoropropoxy)pyrazine, 2-bromo-5-(1,1,1,3,3,3-hexafluoroisopropoxy)pyrazine, 2,5-dichloropyrazine, 2,5-dibromopyrazine, 2-chloro-5-bromopyrazine, and 2-chloro-5-fluoropyrazine.

In the compound represented by formula (1), X is preferably a chlorine atom.

The amount used of the 3-ethylsulfonylpyridine N-oxide is within the range of usually 0.8 to 3 moles, and preferably 1 to 2 moles, as opposed to 1 mole of the compound represented by formula (1).

Specific examples of the compound represented by formula (4) include trimethylphosphine, methyl(di-t-butyl)phosphine, 2-butenyl(di-t-butyl)phosphine, and 3-methyl-2-butenyl(di-t-butyl)phosphine.

Specific examples of the compound represented by formula (5) include trimethylphosphonium tetrafluoroborate, methyl(di-t-butyl)phosphonium tetrafluoroborate, 2-butenyl(di-t-butyl)phosphonium tetrafluoroborate, and 3-methyl-2-butenyl(di-t-butyl)phosphonium tetrafluoroborate.

The amount used of the compound represented by formula (4) or the compound represented by formula (5) is within the range of usually 0.1 to 50 moles, and preferably 0.5 to 20 moles, as opposed to 1 mole of the palladium compound or the nickel compound.

Specific examples of the compound represented by formula (6) include 1,4-bis(di-t-butylphosphino)ethane, 1,4-bis(di-t-butylphoshino)propane, and 1,4-bis(di-t-butylphoshino)butane.

Specific examples of the compound represented by formula (7) include 1,4-bis(di-t-butylphosphino)ethane bistetrafluoroborate, 1,4-bis(di-t-butylphosphino)propane bistetrafluoroborate, and 1,4-bis(di-t-butylphosphino)butane bistetrafluoroborate.

The amount used of the compound represented by formula (6) or the compound represented by formula (7) is within the range of usually 0.05 to 30 moles, and preferably 0.3 to 20 moles, as opposed to 1 mole of the palladium compound or the nickel compound.

Preferred examples of the compound selected from Group P include 2-butenyl(di-t-butyl)phosphine, 2-butenyl(di-t-butyl)phosphonium tetrafluoroborate, methyl(di-t-butyl)phosphine, methyl(di-t-butyl)phosphonium tetrafluoroborate, 1,4-bis(di-t-butylphosphino)butane, and 1,4-bis(di-t-butylphosphino)butane bistetrafluoroborate.

Examples of the base include organic bases and inorganic bases, and the inorganic bases are preferably used. Examples of the inorganic bases include alkali metal hydroxides, alkaline earth metal hydroxides, alkali metal carboxylate salts, alkaline earth metal carboxylate salts, alkali metal carbonate salts, alkaline earth metal carbonate salts, alkali metal bicarbonate salts, alkaline earth metal bicarbonate salts, alkali metal phosphate salts, and alkaline earth metal phosphate salts, and alkali metal carbonate salts and alkali metal phosphate salts are preferably used. More specific examples thereof include lithium hydroxide, sodium hydroxide, potassium hydroxide, cesium hydroxide, calcium hydroxide, barium hydroxide, sodium formate, potassium formate, calcium formate, sodium acetate, potassium acetate, sodium carbonate, potassium carbonate, cesium carbonate, calcium carbonate, sodium bicarbonate, potassium bicarbonate, sodium phosphate, and potassium phosphate, and sodium carbonate, potassium carbonate, cesium carbonate, sodium phosphate, and potassium phosphate are preferred.

The amount used of the base is within the range of usually 1 to 5 moles, and preferably 1.5 to 2.5 moles, as opposed to 1 mole of the compound represented by formula (1) .

Examples of the carboxylic acid include aliphatic carboxylic acids such as acetic acid, propionic acid, butanoic acid, and pivalic acid; aromatic carboxylic acids such as benzoic acid, 4-methoxybenzoic acid, 4-methylbenzoic acid, and 4-nitrobenzoic acid; and palladium carboxylate.

Examples of the carboxylate salts include alkali metal salts such as sodium salts and potassium salts of carboxylic acids, and specific examples thereof include sodium acetate, potassium acetate, sodium propionate, potassium propionate, sodium butanoate, potassium butanoate sodium pivalate, potassium pivalate, sodium benzoate, and potassium benzoate.

When the palladium compound to be used in the reaction is palladium carboxylate, the palladium carboxylate can be used in common as the palladium compound and the carboxylate salt. Accordingly, when the palladium carboxylate salt is used as the palladium compound, the carboxylic acid or the carboxylate salt may be further added to the reaction separately, or may not be added.

When the nickel compound to be used in the reaction is nickel carboxylate salt, the nickel carboxylate salt may be used in common as the nickel compound and the carboxylate salt. Accordingly, when the nickel carboxylate salt is used as the nickel compound, the carboxylic acid or the carboxylate salt may be further added to the reaction separately, or may not be added.

The amount used of the carboxylic acid or the carboxylate salt is within the range of usually 0.1 to 50 moles as opposed to 1 mole of the palladium compound or the nickel compound. When the palladium carboxylate salt is used as the palladium compound, and also the carboxylic acid or the carboxylate salt is further added separately, the total amounts of the amount used of the palladium carboxylate salt and the amount used of the carboxylic acid or the carboxylate salt is within the range of usually 1 to 50 moles as opposed to 1 mole of the palladium compound. When the nickel carboxylate salt is used as the nickel compound, and also the carboxylic acid or the carboxylate salt is further added separately, the total amounts of the amount used of the nickel carboxylate salt and the amount used of the carboxylic acid or the carboxylate salt is within the range of usually 1 to 50 moles as opposed to 1 mole of the nickel compound.

When the carboxylic acid is used in the reaction, the carboxylate salt may be formed by reacting the carboxylic acid with the base. When the carboxylate salt is formed by reacting the carboxylic acid with the base, the formed carboxylate salt may be used as carboxylate salt.

When the carboxylate salt to be used in the reaction is a basic salt, the carboxylate salt may be also used in common as the carboxylate salt and the base. In the case, the amount of the carboxylate salt is the total amounts of the above-mentioned amount used of the base and the above-mentioned amount used of the carboxylic acid or the carboxylate salt.

The reaction may be conducted in the presence of a copper or a copper compound, and is preferably conducted in the presence of the copper or the copper compound.

Examples of the copper include copper powder. Examples of the copper compound include one (1) valent of the copper compound such as copper(I) oxide.

The amount used of the copper or the copper compound is within the range of usually 0.1 to 50 moles and preferably 0.3 to 20 moles, as opposed to 1 mole of the palladium compound or the nickel compound.

The reaction temperature of the reaction is within the range of usually 80 to 180°C and preferably 100 to 150°C.

The reaction period of the reaction is usually within the range of 0.1 to 48 hours.

Embodiments of the reaction should not be limited to specific ones thereof, and the reaction is usually conducted by mixing the compound selected from the Group P, the carboxylic acid or the carboxylate salt, the palladium compound or the nickel compound, the base and the solvent, followed by adding 3-ethylsulfonylpyridine N-oxide and the compound represented by formula (1), and finally as needed by adding the copper or the copper compound.

When the reaction is completed, for example, the resulting mixture is filtered to remove solids such as inorganic salts, and the organic solvents are evaporated; water is added to the resulting mixture to dissolve the solids such as the inorganic salts, and as needed, the mixture solution is diluted by adding the solvents, the aqueous layers are separated, the resulting organic layers are washed with water, and the organic solvents are evaporated to obtain the compound represented by formula (2). The obtained compound may be further purified by crystallization or column chromatography etc.

Also, the compound represented by formula (2) may be supplied to the step (b) without any purification.

Specific examples of the compound represented by formula (2) that may be obtained according to the above-mentioned process include 2-[3-(ethylsulfonyl)-1-oxide-2-pyridinyl]-5-methoxypyrazine, 2-[3-(ethylsulfonyl)-1-oxide-2-pyridinyl]-5-ethoxypyrazine, 2-[3-(ethylsulfonyl)-1-oxide-2-pyridinyl]-5-propoxypyrazine, 2-[3-(ethylsulfonyl)-1-oxide-2-pyridinyl]-5-isopropoxypyrazine, 2-[3-(ethylsulfonyl)-1-oxide-2-pyridinyl]-5-trifluoromethoxypyrazine, 2-[3-(ethylsulfonyl)-1-oxide-2-pyridinyl]-5-(2,2,2-trifluoroethoxy)pyrazine, 2-[3-(ethylsulfonyl)-1-oxide-2-pyridinyl]-5-(1,1,2,2-tetrafluoroethoxy)pyrazine, 2-[3-(ethylsulfonyl)-1-oxide-2-pyridinyl]-5-(2,2,3,3,3-pentafluoropropoxy)pyrazine, 2-[3-(ethylsulfonyl)-1-oxide-2-pyridinyl]-5-(2,2,3,3-tetrafluoropropoxy)pyrazine, 2-[3-(ethylsulfonyl)-1-oxide-2-pyridinyl]-5-(3,3,3-trifluoropropoxy)pyrazine, 2-[3-(ethylsulfonyl)-1-oxide-2-pyridinyl]-5-(1,1,1,3,3,3-hexafluoroisopopoxy)pyrazine, 2-[3-(ethylsulfonyl)-1-oxide-2-pyridinyl]-5-chloropyrazine, 2-[3-(ethylsulfonyl)-1-oxide-2-pyridinyl]-5-bromopyrazine, and 2-[3-(ethylsulfonyl)-1-oxide-2-pyridinyl]-5-fluoropyrazine.

Next, the step (b) is explained.

In the step (b), the compound represented by formula (2) can be subjected to a reduction reaction to obtain a compound represented by formula (3).

The reduction reaction is usually conducted by using hydrogen sources and reduction catalyst.

Examples of the hydrogen sources include hydrogen gas, formic acid and formate salt and the others, and hydrogen gas is preferred. Examples of the formate salts include sodium formate, potassium formate, and ammonium formate.

The amount used of the hydrogen gas is usually within the range of 1 to 20 moles as opposed to 1 mole of the compound represented by formula (2).

Examples of the reduction catalyst include noble metals-supported catalysts in which noble metals such as palladium, platinum, iridium, and rhodium and the like are supported on carriers such as activated carbon, alumina, and silica; and Raney nickel.

The amount used of the reduction catalyst is usually within the range of 0.0001 to 0.5 moles as opposed to 1 mole of the compound represented by formula (2).

The above-mentioned carriers are added to the products that are obtained from the palladium compound to be used in the step (a) to obtain the resulting mixture, which can be used as a reduction catalyst. Specifically, the reaction can be conducted by adding the hydrogen sources and the above-mentioned carriers to the reaction mixture obtained in step (a), said mixture comprising the products obtained from the compounds represented by formula (2) and the palladium compound. In this case, the amount used of the carriers is within the range of 0.005 to 1 parts by weight as opposed to 1 part by weight of the compound represented by formula (1).

The reaction is usually conducted in a solvent. Examples of the solvents include ethers such as methyl t-butyl ether, dibutyl ether, and tetrahydrofuran; aromatic hydrocarbons such as toluene and xylene; and aliphatic hydrocarbons such as hexane and heptane; and mixture of two or more kinds thereof. When the reaction is conducted by using the reaction mixture obtained in the step (a), the solvents contained in the reaction mixture may be used as they are.

The amount used of the solvents is usually within the range of 1 to 50 parts by weight as opposed to 1 part by weight of the compound represented by formula (2).

The reaction temperature of the reaction is within the range of usually 0 to 120°C and preferably 20 to 100°C.

The reduction reaction may be conducted under atmospheric pressure condition, or may be conducted under pressure condition.

When the reduction reaction is conducted under pressure condition with hydrogen gas, the degree of the pressure is usually within the range of 0.1 to 1.5 MPa.

The reaction period of the reaction is within the range of 0.1 to 48 hours.

Embodiments of the reaction should not be limited to specific ones thereof, and the reaction is usually conducted by mixing the compound represented by formula (2), the hydrogen sources, the reduction catalysts and the solvents. Specifically, the reduction reaction is conducted by mixing the compound represented by formula (2), the reduction catalysts and the solvents, followed by subjecting the reaction system to a hydrogen gas atmosphere; the compound represented by formula (2), the reduction catalyst and the solvents are mixed, followed by adding formic acid or formate salt.

When the reduction reaction is conducted by using the reaction mixture obtained in the step (a), the reaction is conducted by adding the carriers to the reaction mixture obtained in the step (a), followed by subjecting the reaction system to a hydrogen atmosphere; and followed by adding the carriers to the reaction mixture obtained in the step (a), and thereafter by adding formic acid or formate salt.

When the reaction is completed, the resulting mixture is filtered to remove the solids containing the noble metals-supported catalysts, and the filtrates are then concentrated; any procedures such as an addition of acidic aqueous solutions such as hydrochloric acid and sulfuric acid to the resulting mixture so as to dissolve Raney nickel etc., are conducted, and as needed, the solvents are added to the mixture to dilute the resulting mixture, the aqueous layers are separated, the resulting organic layers are washed with water, and then the organic solvents are evaporated to obtain the compound represented by formula (3). The obtained compound can be further purified by crystallization or column chromatography etc.

Specific examples of the compounds represented by formula (3) that may be obtained according to the process include 2-[3-(ethylsulfonyl)-2-pyridinyl]-5-methoxypyrazine, 2-[3-(ethylsulfonyl)-2-pyridinyl]-5-ethoxypyrazine, 2-[3-(ethylsulfonyl)-2-pyridinyl]-5-n-propoxypyrazine, 2-[3-(ethylsulfonyl)-2-pyridinyl]-5-isopropoxypyrazine, 2-[3-(ethylsulfonyl)-2-pyridinyl]-5-trifluoromethoxypyrazine, 2-[3-(ethylsulfonyl)-2-pyridinyl]-5-(2,2,2-trifluoroethoxy)pyrazine, 2-[3-(ethylsulfonyl)-2-pyridinyl]-5-(1,1,2,2-tetrafluoroethoxy)pyrazine, 2-[3-(ethylsulfonyl)-2-pyridinyl]-5-(2,2,3,3,3-pentafluoropropoxy)pyrazine, 2-[3-(ethylsulfonyl)-2-pyridinyl]-5-(2,2,3,3-tetrafluoropropoxy)pyrazine, 2-[3-(ethylsulfonyl)-2-pyridinyl]-5-(3,3,3-trifluoropropoxy)pyrazine, 2-[3-(ethylsulfonyl)-2-pyridinyl]-5-(1,1,1,3,3,3-hexafluoroisopopoxy)pyrazine, 2-[3-(ethylsulfonyl)-2-pyridinyl]-5-chloropyrazine, 2-[3-(ethylsulfonyl)-2-pyridinyl]-5-bromopyrazine, and 2-[3-(ethylsulfonyl)-2-pyridinyl]-5-fluoropyrazine.

### [Process for preparing 3-ethylsulfonylpyridine N-oxide]

3-Ethylsulfonylpyridine N-oxide can be obtained by reacting 3-ethylsulfonylpyridine with an oxidizing agent.

The reaction is usually conducted in a solvent. Examples of the solvents include halogenated hydrocarbons such as chloroform and methylene chloride; nitrile solvents such as acetonitrile and propionitrile; acetic acid; water; and mixture of two or more kinds thereof.

Examples of the oxidizing agent include sodium periodate, 3-chloroperbenzoic acid, and hydrogen peroxide.

When the hydrogen peroxide is used as an oxidizing agent, as needed, sodium carbonate and a catalyst may be added to the reaction. Examples of the catalyst include tungsten acid and sodium tungstate.

The amount used of the oxidizing agent is usually within the range of 1 to 10 moles as opposed to 1 mole of 3-ethylsulfonylpyridine.

When the reaction is conducted by adding sodium carbonate, the amount used of the sodium carbonate is usually within the range of 0.01 to 1 mole(s) as opposed to 1 mole of 3-ethylsulfonylpyridine. When the reaction is conducted by adding the catalyst, the amount used of the catalyst is usually within the range of 0.01 to 0.5 moles as opposed to 1 mole of 3-ethylsulfonylpyridine.

The reaction period of the reaction is usually within the range of 5 minutes to 24 hours. The reaction temperature of the reaction is usually within the range of -20°C to 80°C.

3-Ethylsulfonylpyridine is a commercially available compound.

When the reaction is completed, water is added to the reaction mixture, and the mixture is extracted with organic solvents, and as needed, the organic layers are washed with an aqueous solution of the reducing agent (for example, sodium sulfite, and sodium thiosulfate) etc., and the resulting organic layers are dried and concentrated to isolate 3-ethylsulfonylpyridine N-oxide.

### [Process for preparing the compound represented by formula (1)]

The compound represented by formula (1) may be obtained by reacting 2,5-dichloropyrazine or 2,5-dibromopyrazine with a compound represented by formula (8):

Q-OH (8)

(wherein Q is the same as defined above.)
in the presence of a base.

The reaction may be conducted in a solvent, or may be conducted in the absence of any solvents. Examples of the solvents include ethers such as tetrahydrofuran, methyl t-butyl ether, and ethylene glycol dimethyl ether; aromatic hydrocarbons such as toluene and xylene; nitriles such as acetonitrile and propionitrile; and mixture of two or more kinds thereof.

Examples of the base include alkali metal hydrides such as lithium hydride and sodium hydride; organic bases such as triethylamine and diisopropylethylamine; alkaline metal carbonates such as sodium carbonate and potassium carbonate.

The amount used of the compound represented by formula (8) is usually within the range of 1 to 10 mole(s) as opposed to 1 mole of 2,5-dichloropyrazine or 2,5-dibromopyrazine.

The amount used of the base is usually within the range of 1 to 5 mole(s) as opposed to 1 mole of 2,5-dichloropyrazine or 2,5-dibromopyrazine.

The reaction temperature of the reaction is usually within the range of -20°C to 150°C. The reaction period of the reaction is usually within the range of 0.5 to 24 hours.

2,5-Dichloropyrazne, 2,5-dibromopyrazine and the compound represented by formula (8) are commercially available compounds or can be prepared by a publically known method.

When the reaction is completed, water is added to the reaction mixture, and the mixture is extracted with organic solvents, and the organic layers are worked up (for example, drying and concentration) to obtain the compound represented by formula (1).

### EXAMPLES

Hereinafter, the present invention is explained in more detail by using Examples etc., however, the present invention should not be limited to these examples.

In the below-mentioned examples, unless otherwise specified, the quantitative analysis was conducted with gas chromatography (which is hereinafter referred to as GC). The analysis condition is as follows.

### [Measurement Condition of Gas Chromatography (GC))]

· Measurement Instrument: manufactured by Shimazu GC-2010
· Column: DB-1 length 30 m, internal diameter 250 µm, coating thickness 1.00 µm (manufactured by Agilent Technologies)
· Column Temperature: raised from 50°C to 300°C in a ratio of 10°C/min, and then kept at 300°C for 10 minutes
· Helium Gas Flow Rate: 1.0 mL/min
· Internal Standard Substance: 4-Dimethlyamino toluene
· Injected amount: 1 µL

### (Example 1)

Under nitrogen atmosphere, in 100 mL Schlenk tube, palladium(II) chloride 3.4 mg, 2-butenyl(di-t-butyl) phosphonium tetrafluoroborate 9.1 mg, sodium acetate 3.1 mg, and potassium carbonate 110 mg, and toluene 1 g were mixed, and the mixture was stirred at 120°C for 10 minutes. To the resulting mixture was added 3-ethylsulfonylpyridine N-oxide 87 mg and the mixture was stirred at 120°C for 10 minutes. Separately, 2-chloro-5-(2,2,3,3,3-pentafluoropropoxy)pyrazine 100 mg was diluted with toluene 1 g, and thereafter, the diluted solutions were added to the reaction mixture, and the mixture was further stirred at 120°C for 8 hours.

The resulting reaction mixture was cooled to room temperature, and the mixture was analyzed with gas chromatography to find that the 3-ethylsulfonylpyridine N-oxide was remained in 30% (GC area percentage method). Also the reaction mixture was analyzed with gas chromatography internal standard method to find that 2-[3-(ethylsulfonyl)-1-oxide-2-pyridinyl]-5-(2,2,3,3,3-pentafluoropropoxy)pyrazine was produced in 52 % yields and 2-[3-(ethylsulfonyl)-2-pyridinyl]-5-(2,2,3,3,3-pentafluoropropoxy)pyrazine was produced in 10 % yields.

The reaction mixture was transferred to an autoclave with glass inner tube, and activated carbon 40 mg was added thereto, and the atmosphere in the autoclave was purged with hydrogen gas. The mixture was stirred at 90°C under hydrogen pressure of 1 MPa for 6 hours. The resulting mixture was cooled to room temperature, and thereafter, analyzed with gas chromatography inner standard method to find that all of 2-[3-(ethylsulfonyl)-1-oxide-2-pyridinyl]-5-(2,2,3,3,3-pentafluoropropoxy)pyrazine was converted into 2-[3-(ethylsulfonyl)-2-pyridinyl]-5-(2,2,3,3,3-pentafluoropropoxy)pyrazine and the yields of 2-[3-(ethylsulfonyl)-2-pyridinyl]-5-(2,2,3,3,3-pentafluoropropoxy)pyrazine was 61 % yields (total yields of two steps of the coupling step and the reduction step).

### (Example 2)

2-[3-(Ethylsulfonyl)-2-pyridinyl]-5-(2,2,3,3,3-pentafluoropropoxy)pyrazine that was isolated by silica gel chromatography was white crystals, and its melting point was 87.9°C.

### (Example 3)

Under nitrogen atmosphere, in 100 mL Schlenk tube, palladium(II) chloride 2 mg, 2-butenyl(di-t-butyl) phosphonium tetrafluoroborate 5.5 mg, copper(I) oxide 20 mg, sodium acetate 3.1 mg, and potassium carbonate 110 mg, and toluene 1 g were mixed, and the mixture was stirred at 120°C for 10 minutes. To the resulting mixture was added 3-ethylsulfonylpyridine N-oxide 87 mg and the mixture was stirred at 120°C for 10 minutes. Separately, 2-chloro-5-(2,2,3,3,3-pentafluoropropoxy)pyrazine 100 mg was diluted with toluene 1 g, and thereafter, the diluted solutions were added to the reaction mixture, and the mixture was further stirred at 120°C for 8 hours.

The resulting reaction mixture was cooled to room temperature, and the mixture was analyzed with gas chromatography to find that the 3-ethylsulfonylpyridine N-oxide was remained in 45% (GC area percentage method). Also the reaction mixture was analyzed with gas chromatography internal standard method to find that 2-[3-(ethylsulfonyl)-1-oxide-2-ppyridinyl]-5-(2,2,3,3,3-pentafluoropropoxy)pyrazine was produced in 45 % yields and 2-[3-(ethylsulfonyl)-2-pyridinyl]-5-(2,2,3,3,3-pentafluoropropoxy)pyrazine was produced in 11 % yields.

### (Example 3)

2-[3-(Ethylsulfonyl)-2-pyridinyl]-5-(2,2,3,3,3-pentafluoropropoxy)pyrazine
¹H-NMR (CDCl₃, TMS) δ: 1.29(3H, t), 3.40(2H, q), 4.89(2H, td), 7.53(1H, dd), 7.94(1H, d), 8.40(1H, d), 8.51(2H, dd). GC-MS: [M⁺] 413

### (Example 4)

To a test tube with lid were added palladium(II) chloride 1 mg, 1,4-bis(di-t-butylphosphino)butane bistetrafluoroborate 3.0 mg, benzoic acid 2 mg, copper(I) oxide 1.4 mg, potassium carbonate 54 mg, ethylsulfonylpyridine N-oxide 44 mg, 2-chloro-5-(2,2,3,3,3-pentafluoropropoxy)pyrazine 50 mg, and toluene 1 g, and the atmosphere of the reaction system was replaced with nitrogen gas, and the mixture was stirred at 120°C for 8 hours.

After the resulting reaction mixture was cooled to room temperature, the mixture was analyzed with gas chromatography to find that 3-ethylsulfonylpyridine N-oxide was remained in 12.5 % (GC area percentage method). Also the reaction mixture was analyzed with gas chromatography internal standard method to find that 2-[3-(ethylsulfonyl)-1-oxide-2-pyridinyl]-5-(2,2,3,3,3-pentafluoropropoxy)pyrazine was produced in 56 % yields, and 2-[3-(ethylsulfonyl)-2-pyridinyl]-5-(2,2,3,3,3-pentafluoropropoxy)pyrazine was produced in 1 % yield.

### (Example 5)

Under nitrogen atmosphere, in 100 mL Schlenk tube, palladium(II) chloride 3.6 mg, methyl(di-t-butyl) phosphonium tetrafluoroborate 8.4 mg, copper(I) oxide 10 mg, sodium acetate 5.0 mg, and potassium carbonate 190 mg, and toluene 1 g were mixed, and the mixture was stirred at 120°C for 10 minutes. To the resulting mixture was added 3-ethylsulfonylpyridine N-oxide 150 mg and the mixture was stirred at 120°C for 10 minutes. Separately, 2-dichloropyrazine 100 mg was diluted with toluene 1 g, and thereafter, the diluted solutions were added to the reaction mixture, and the mixture was further stirred at 120°C for 8 hours.

The resulting reaction mixture was cooled to room temperature, and the mixture was analyzed with gas chromatography to find that 3-ethylsulfonylpyridine N-oxide was remained in 49% (GC area percentage method). Also the reaction mixture was analyzed with gas chromatography internal standard method to find that 2-[3-(ethylsulfonyl)-1-oxide-2-pyridinyl]-5-chloropyrazine was produced in 36 % yields and 2-[3-(ethylsulfonyl)-2-pyridinyl]-5-chloropyrazine was produced in 15 % yields.
2-[3-(Ethylsulfonyl)-2-pyridinyl]-5-chloropyrazine
GC-MS: [M⁺] 299

### (Example 6)

To a test tube with lid were added nickel(II) chloride 2.5 mg, 1M trimethylphosphine solution in toluene 0.13 mL, and benzoic acid 2.8 mg, copper(I) oxide 4.2 mg, potassium carbonate 54 mg, 3-ethylsulfonylpyridine N-oxide 44 mg, 2-chloro-5-(2,2,3,3,3-pentafluoropropoxy)pyrazine 50 mg, and toluene 1 g, and the atmosphere of the reaction system was replaced with nitrogen gas, and the mixture was stirred at 120°C for 8 hours.

After the resulting reaction mixture was cooled to room temperature, the mixture was analyzed with gas chromatography to find that 3-ethylsulfonylpyridine N-oxide was remained in 48 % (GC area percentage method). Also the reaction mixture was analyzed with gas chromatography internal standard method to find that 2-[3-(ethylsulfonyl)-1-oxide-2-pyridinyl]-5-(2,2,3,3,3-pentafluoropropoxy)pyrazine was produced in 44 % yields, and 2-[3-(ethylsulfonyl)-2-pyridinyl]-5-(2,2,3,3,3-pentafluoropropoxy)pyrazine was produced in 4 % yields.

### (Example 7)

The reaction was conducted similarly to the method described in Example 6 except that 1M trimethylphoshine solution in toluene 0.25 mL was used.

The resulting reaction mixture was cooled to room temperature, and the mixture was analyzed with gas chromatography to find that 3-ethylsulfonylpyridine N-oxide was remained in 20 % (GC area percentage method). Also the reaction mixture was analyzed with gas chromatography internal standard method to find that 2-[3-(ethylsulfonyl)-1-oxide-2-pyridinyl]-5-(2,2,3,3,3-pentafluoropropoxy)pyrazine was produced in 53 % yields, and 2-[3-(ethylsulfonyl)-2-pyridinyl]-5-(2,2,3,3,3-pentafluoropropoxy)pyrazine was produced in 6 % yields.

### (Example 8)

Under nitrogen atmosphere, in 100 mL Schlenk tube, palladium(II) acetate 13.3 mg, methyl(di-t-butyl) phosphonium tetrafluoroborate 34.8 mg, sodium acetate 31.9 mg, potassium carbonate 547 mg, and toluene 2 g were mixed, and the mixture was stirred at 120°C for 10 minutes. To the resulting mixture was added 3-ethylsulfonylpyridine N-oxide 489 mg and the mixture was stirred at 120°C for 10 minutes. Separately, 2-dichloropyrazine 587 mg was diluted with toluene 1 g, and thereafter, the diluted solutions were added to the reaction mixture, and the mixture was further stirred at 120°C for 6 hours.

The resulting reaction mixture was cooled to room temperature, and the mixture was analyzed with gas chromatography to find that 3-ethylsulfonylpyridine N-oxide was remained in 20% (GC area percentage method). Also the reaction mixture was analyzed with gas chromatography internal standard method to find that 2-[3-(ethylsulfonyl)-1-oxide-2-pyridinyl]-5-(2,2,3,3,3-pentafluoropropoxy)pyrazine was produced in 65 % yields and 2-[3-(ethylsulfonyl)-2-pyridinyl]-5-(2,2,3,3,3-pentafluoropropoxy)pyrazine was produced in 5 % yields.

Water 10 g was added to the reaction mixture, and the mixture was extracted with ethyl acetate 10 mL twice and the mixture was dried over magnesium sulfate, and the solvents were evaporated to obtain yellow brown solids. The solids were subjected to silica gel column to fractionally obtain as impurity products, an isomer in which 3-ethylsulfonylpyridine N-oxide was coupled at the 6-position thereof, and an isomer in which 3-ethylsulfonylpyridine N-oxide was coupled at the 2- and 6-positions thereof, and these products were identified. 2-[3-(Ethylsulfonyl)-1-oxide-6-pyridinyl] -5-(2,2,3,3,3-pentafluoropropoxy)pyrazine
¹H-NMR (CDCl₃, TMS) δ: 1.38(3H, t), 3.23(2H, q), 4.96(2H, td), 7.74(1H, dd), 8.48(1H, d), 8.53(1H, d), 8.79(1H, d), 10.06(1H, d).
GC-MS: [M⁺] 413
2,2'-[3-(Ethylsulfonyl)-1-oxide-2,6-pyridinyl]-bis[5-(2,2,3,3,3-pentafluoropropoxy)pyrazine]
¹H-NMR (CDCl₃, TMS) δ: 1.32(3H, t), 3.33(2H, q), 4.93(4H, td), 8.05(1H, d), 8.46(1H, d), 8.49(2H, t), 8.61(1H, d), 9.93(1H, d).
GC-MS: [M⁺] 639

### (Reference Example 1)

### Process-1 for preparing 3-ethylsulfonylpyridine N-oxide

3-Ethylsulfonylpyridine 17.1 g and 3-chloroperbenzoic acid (containing 30 % water) 27.1 g were added to chloroform 170 g, and the mixture was stirred at room temperature for 1 hour. The participated slurry was filtered, and the filtrates were washed with 20% aqueous sodium sulfite solution 100 mL, and then concentrated under reduced pressure. The resulting residues were purified by silica gel column chromatography to obtain 3-ethylsulfonylpyridine N-oxide 11.6 g (62% yields).
3-Ethylsulfonylpyridine N-oxide
¹H-NMR (CDCl₃, TMS) δ: 1.35(3H, t, J=7.2Hz), 3.21(2H, q, J=7.2Hz), 7.49-7.52(1H, m), 7.70-7.72(1H, m), 8.40-8.42(1H, m), 8.67-8.68(1H, m).

### (Reference Example 2)

### Process-2 for preparing 3-ethylsulfonylpyridine N-oxide

In 300 mL round-bottom flask with a stirrer, to sodium tungstate 4.27 g and water 10 g was added 30% aqueous hydrogen peroxide 5 g, and then sulfuric acid 1.3 g was further added. To the resulting mixture was added 3-ethylsulfonylpyridine 74 g, and to the mixture was added dropwise 30% aqueous hydrogen peroxide solution 75 g over 1 hour while the mixture was stirred at 90°C. After the dropwise addition, the mixture was stirred at 90°C for 4 hours.

The resulting reaction mixture was cooled to room temperature, and the mixture was analyzed with gas chromatography area percentage method to find that 3-ethylsulfonylpyridine N-oxide was produced in 97 % yields, and also 3-ethylsulfonylpyridine was produced in 3 % yields. The reaction mixture was extracted with ethyl acetate 150 g three times. The resulting organic layers were combined, and the combined organic layers were washed with 20% aqueous sodium sulfite solution 100 mL, and dried over magnesium sulfate, and concentrated under reduced pressure. The resulting residue was recrystallized from isopropyl alcohol to obtain 3-ethylsulfonylpyridine N-oxide 35 g as white crystal.

### Industrial Applicability

The process of the present invention can provide an intermediate compound for preparing 2-substituted-3-ethylsulfonylpyridine as an active ingredient of pest control agent, and 2-substituted-3-ethylsulfonylpyridine compound.

## Claims

1. A process for preparing a compound represented by the below-mentioned formula (2), which comprises a step (a) : a step of reacting of 3-ethylsulfonylpyridine N-oxide with a compound represented by formula (1): 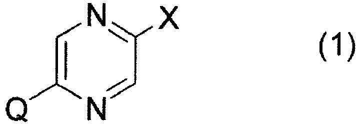 (wherein X represents a chlorine atom or a bromine atom, Q represents a C1-3 alkoxy group optionally substituted with one or more fluorine atoms, a fluorine atom, a chlorine atom, or a bromine atom.)
in the presence of one or more compounds selected from Group P, a carboxylic acid or a carboxylate salt, a base, and a palladium compound or a nickel compound in a solvent to obtain a compound represented by formula (2) : 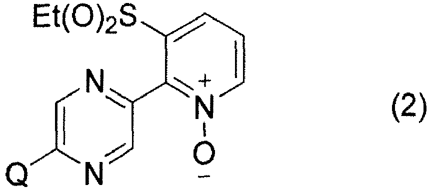 (wherein Q is the same as defined above.),
Group P: a group consisting of the following compounds:
a compound represented by formula (4):
R₃P (4)
(wherein R represents an alkyl group having 1 to 6 carbon atoms or an alkenyl group having 3 to 6 carbon atoms),
a compound represented by formula (5):
R₃PH⁺Z⁻ (5)
(wherein R is the same as defined above, and Z⁻ represents an anion.),
a compound represented by formula (6):
R₂P(CH₂)ₙPR₂ (6)
(wherein R is the same as defined above, and n is an integer of 1 to 4.), and
a compound represented by formula (7):
R₂PH⁺(CH₂)ₙPR₂H⁺2Z⁻ (7)
(wherein R, n and Z⁻ are the same as defined above.).

2. The process according to claim 1 wherein the compound selected from Group P is one or more compounds selected from 2-butenyl(di-t-butyl)phosphine, trimethylphosphine, 2-butenyl(di-t-butyl)phosphonium tetrafluoroborate, methyl(di-t-butyl)phosphine, methyl(di-t-butyl)phosphonium tetrafluoroborate, trimethylphosphonium tetrafluoroborate, 1,4-bis(di-t-butylphosphino)butane, or 1,4-bis(di-t-butylphosphino)butane bistetrafluoroborate.

3. The process according to claim 1 or 2 wherein the step (a) is conducted in the presence of copper or copper compound.

4. The process according to claim 3 wherein the copper is copper powder, and the copper compound is copper(I) oxide.

5. A process for preparing a compound represented by the below-mentioned formula (3): 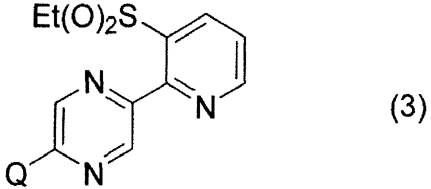 (wherein Q represents a C1-3 alkoxy group optionally substituted with one or more fluorine atoms, a fluorine atom, a chlorine atom, or a bromine atom.)
, which comprises a step (a) according to any one of claims 1 to 4 and the below-mentioned step (b),
a step (b): a step of subjecting the compound represented by formula (2): 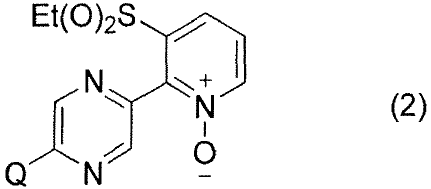 (wherein Q is the same as defined above.)
to a reduction reaction to obtain the compound represented by formula (3).

6. The process according to claim 5 wherein in the step (b), the reduction reaction is conducted by using hydrogen gas.

7. The process according to any one of claims 1 to 6 wherein X represents a chlorine atom, and Q represents a 2,2,3,3,3-pentafluoropropoxy group.

8. A 3-ethylsulfonylpyridine N-oxide.

9. A compound represented by formula (2): 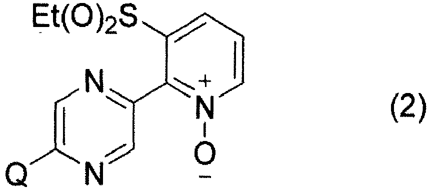 (wherein Q represents a C1-3 alkoxy group optionally substituted with one or more fluorine atoms, a fluorine atom, a chlorine atom, or a bromine atom.).

10. A 2-[3-(ethylsulfonyl)-1-oxide-2-pyridinyl]-5-(2,2,3,3,3-pentafluoropropoxy)pyrazine.

11. A 2-[3-(ethylsulfonyl)-1-oxide-2-pyridinyl]-5-chloropyrazine.
